(19) Europäisches Patentamt
European Patent Office
Office européen des brevets

(11) Numéro de publication: **0 289 416**
**A1**

(12) # DEMANDE DE BREVET EUROPEEN

(21) Numéro de dépôt: **88401016.6**

(22) Date de dépôt: **26.04.88**

(51) Int. Cl.[4]: **C 12 P 9/00**
C 12 N 9/16

(30) Priorité: **28.04.87 FR 8705994**

(43) Date de publication de la demande:
**02.11.88 Bulletin 88/44**

(84) Etats contractants désignés:
**AT BE CH DE ES FR GB GR IT LI LU NL SE**

(71) Demandeur: **BIOEUROPE**
**4 Impasse Didier Daurat**
**F-31400 Toulouse (FR)**

(72) Inventeur: **Pradines, Antoine**
**18 Avenue Aristide Briand**
**F-31400 Toulouse (FR)**

**Klaebe, Alain**
**Avenue des Pyrénnées Vigoulet-Auzil**
**F-31320 Castanet (FR)**

**Perie, Pierre**
**Le Catilat Vigoulet-Auzil**
**F-31320 Castanet (FR)**

**Monsan, Pierre**
**Renoufail Mondonville**
**F-31700 Blagnac (FR)**

(74) Mandataire: **Colas, Jean-Pierre et al**
**Cabinet de Boisse 37, avenue Franklin D. Roosevelt**
**F-75008 Paris (FR)**

(54) **Procédé de phosphorylation enzymatique de composés organiques hydroxylés à l'aide de phosphatase alcaline d'intestin de veau.**

(57) L'invention se rapporte aux biotechnologies.

Elle concerne un procédé de préparation de dérivés phosphatés de composés hydroxylés caractérisé en ce qu'on effectue une réaction de phosphorylation enzymatique mettant en jeu un composé organique hydroxylé, un donneur de phosphate qui est un pyrophosphate ou polyphosphate de la formule :

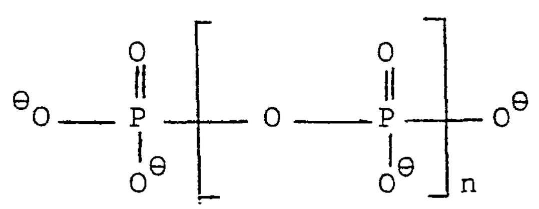

où n = 0 à 74, et
de la phosphatase alcaline d'intestin de veau, à un pH de 4,5 à 9.

Application notamment à la production de α-glycérol phosphate.

EP 0 289 416 A1

**Description**

**Procédé de phosphorylation enzymatique de composés organiques hydroxylés à l'aide de phosphatase alcaline d'intestin de veau.**

L'invention concerne un procédé de phosphorylation enzymatique de composés organiques hydroxylés à l'aide de phosphatase alcaline d'intestin de veau et les composés phosphorylés ainsi obtenus.

Diverses phosphorylations enzymatiques ont déjà été réalisés à l'aide de phosphatase alcaline.

Ainsi, Martinek et col.. [Bioorg. Khim., 3, 696 (1977)] ont décrit la synthèse de glycérophosphate à partir de glycérol et de phosphate de tétrabutyl ammonium avec un rendement de l'ordre de 30% en utilisant de la phosphatase alcaline d'intestins de poulets à 240 unités/ml, fixée sur des fibres creuses, en milieu biphasique (eau/chloroforme).

De leur côté, W.B. Anderson et R.C. Nordlie [The Journal of Biological Chemistry, vol. 242, N°1, pages 114-119 (1967)] ont étudié la phosphorylation du glucose à l'aide de phosphatase alcaline purifiée d'Escherichia Coli en utilisant un pyrophosphate comme donneur. Le rendement de transfert de phosphate n'est, cependant, que de 9% pour 91% d'hydrolyse, de sorte que le procédé décrit est peu attractif, sur le plan industriel.

La demanderesse a maintenant trouvé, de façon surprenante, qu'il était possible de synthétiser avec un bon rendement des dérivés phosphatés de certains composés hydroxylés en utilisant de la phosphatase alcaline d'intestin de veau.

Plus particulièrement, l'invention concerne un procédé de préparation de dérivés phosphatés de composés hydroxylés caractérisé en ce qu'on effectue une réaction de phosphorylation enzymatique mettant en jeu un composé organique hydroxylé, un donneur de phosphate qui est un phosphate, un pyrophosphate ou un polyphosphate de la formule

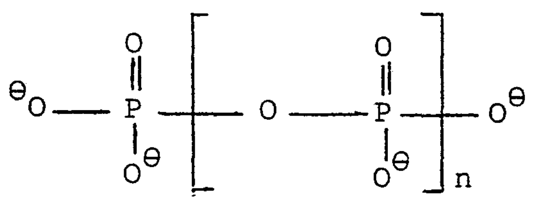

où n = 0 à 74, et
de la phosphatase alcaline d'intestin de veau, à un pH de 4,5 à 9.

Dans le procédé de l'invention, la phosphatase alcaline d'intestin de veau (E.C.3.1.3.1), une hydrolase de monoesters phosphoriques, fonctionne en réversion.

Un des avantages de l'invention est que la phosphatase alcaline extraite de l'intestin de veau n'a pas besoin d'être hautement purifiée. On peut ainsi utiliser de la phosphatase alcaline extraite de l'intestin de veau de qualité courante ayant une activité de l'ordre de 10-30 unités/ml. Une telle phosphatase alcaline est disponible dans le commerce, par exemple le produit commercial SIGMA E.C.3.1.3.1., ref. P7640. Bien entendu, on peut aussi utiliser une phosphatase alcaline d'activité supérieure, si on le désire, bien que cela ne fournisse pas nécessairement de meilleurs résultats.

La réaction de l'invention peut être conduite avec de très nombreux composés organiques hydroxylés, tels que des alcools, des diols, des polyols, des oses, des dérivés d'oses, des peptides et des protéines. Il faut veiller, cependant, à ce que le composé hydroxylé ne contienne pas de groupes réactifs susceptibles d'inhiber l'enzyme, tels que les amino-alcools, ou soit de façon inhérente insensible à la phosphorylation, comme les thiols. Des essais de routines simples permettent de déterminer si un composé hydroxylé donné est susceptible d'être phosphorylé par le procédé de l'invention. La concentration du composé hydroxylé dans le milieu réactionnel peut varier largement mais on préfère des concentrations de 40 à 70% en volume par rapport au mélange réactionnel total.

La réaction doit être conduite dans une gamme de pH où l'enzyme est active, c'est-à-dire à un pH ni trop acide, ni trop basique. On a trouvé que la gamme de pH allant de 4,5 à 9 convient habituellement, les meilleurs résultats étant usuellement obtenus à un pH de 5,3 à 8,0.

La réaction doit aussi être conduite à une température n'affectant pas l'activité de l'enzyme. Des températures dans la gamme de 15 à 50° sont utilisables, la préférence étant donnée à des températures de 30 à 40°.

La durée de la réaction pourra varier largement selon les conditions opératoires, la nature continue ou discontinue du procédé, le type de composé hydroxylé mis en réaction, etc... Elle sera habituellement comprise, toutefois, entre 1 et 100 heures environ.

La quantité de phosphate, de pyrophosphate ou polyphosphate à employer n'est pas critique pourvu qu'il y en ait suffisamment pour la phosphorylation désirée.

La proportion d'enzymes peut aussi varier largement. A titre indicatif on peut utiliser aussi peu que 5 unités

d'activité d'enzyme par millilitre de milieu réactionnel jusqu'à 100 unités et plus. Il n'y a, toutefois, pas d'avantages à utiliser une quantité excessive d'enzyme.

Les exemples non limitatifs suivants sont donnés dans le but d'illustrer le procédé de l'invention :

Exemple 1 Préparation de α-glycérophosphate à partir du glycérol.

On a laissé réagir, dans un tube, pendant 48 heures à 37°C sous agitation lente, 3 ml d'un milieu réactionnel aqueux contenant 55% en volume de glycérol, 100 mg de pyrophosphate de sodium (en abrégé PPi) et 30 mg de phosphatase alcaline d'intestin de veau d'une activité de 1-3 U/mg, le pH du milieu étant réglé à une valeur comprise dans la gamme de 3,7 à 9,90 à l'aide d'une solution-tampon appropriée à laquelle était ajouté du $MgCl_2$, $6H_2O$, 5 millimolaire.

Au bout de 48 heures de réaction, on ultrafiltre le milieu réactionnel pour séparer l'enzyme, on passe le filtrat sur une résine échangeuse d'anions DEAE Sephadex A-25 Pharmacia.

On effectue d'abord un lavage de la résine avec $H_2O$ pour permettre l'élimination du glycérol qui n'est pas retenu sur la résine alors que l'ester et le phosphate restent fixés.

On soumet ensuite la résine à une élution en utilisant, comme éluant, du tampon volatil au bicarbonate de triéthylammonium (TEAB) qui s'élimine facilement par évaporation sous vide, et qui permet d'obtenir l'ester phosphorique et le phosphate sous forme de sel de triéthylammonium.

L'élution se fait à pH 8,8 en utilisant un gradient de concentration en tampon de 150 à 250 mM en TEAB. Les premières fractions contiennent essentiellement l'ester phosphorique alors que les fractions suivantes contiennent un mélange de phosphate et d'ester.

Une étude par $RMN^{31P}$ faite sur ces premières fractions permet de constater la présence des isomères α- et β-glycérol-phosphate, mais aussi celle de deux autres composés phosphorylés présents en très faible concentration non encore identifiés.

On précipite le pyrophosphate et les impuretés en augmentant le pH du milieu réactionnel jusqu'à 10 par addition d'ammoniaque, la précipitation se faisant à une température voisine de 5°C au bout d'une dizaine d'heures.

Le surnageant contient le produit désiré, à savoir l'isomère α-glycérol phosphate.

On a déterminé pour chaque valeur de pH utilisée, le pourcentage de pyrophosphate (PPi) consommé, le rendement d'hydrolyse et le rendement de transfert de phosphate à partir du PPi, ces rendements étant calculés sur la base du % de PPi consommé. Le rendement d'hydrolyse est fonction de l'activité hydrolase de l'enzyme tandis que le rendement de transfert est fonction de l'activité phosphotransférase de l'enzyme. Il est, bien sûr, avantageux d'avoir le rendement de transfert le plus élevé possible. Le tableau suivant résume les résultats obtenus pour les diverses valeurs de pH essayées.

| pH | % de PPi consommé | Rendement d'hydrolyse | Rendement de transfert |
|---|---|---|---|
| 3,7 | 4,3% | 4,3% | 0,0% |
| 4,5 | 70,0% | 48,0% | 22,0% |
| 5,38 | 100,0% | 56,4% | 43,6% |
| 6,40 | 100,0% | 53,6% | 46,4% |
| 6,82 | 100,0% | 43,9% | 56,1% |
| 7,95 | 100,0% | 44,0% | 56,0% |
| 8,94 | 78,7% | 44,4% | 34,3% |
| 9,90 | 10,6% | 5,5% | 5,1% |

On voit qu'on obtient de bons résultats pour des pH allant de 4,5 à environ 9, en particulier entre 5,3 et 8 environ, l'optimum se situant entre 6,8 et 8 environ.

Exemple 2 Comparaison des activités de diverses phosphatases alcalines.

Afin de mettre en évidence la haute activité de la phosphatase alcaline d'intestin de veau comparée à celles de deux phosphatases alcalines d'origine différente, on a réalisé une étude cinétique comparative de la réaction de phosphorylation du glycérol. Pour tous les essais réalisés, on a utilisé un milieu réactionnel d'un volume total de 3 ml contenant 60% en volume de glycérol, 100 mg de pyrophosphate de sodium, 350 unités de phosphatase alcaline, le pH étant réglé à 7,9 à l'aide d'une solution tampon appropriée à laquelle était ajouté du $MgCl_2$, $6H_2O$, 5 millimolaire. Le suivi de la réaction est effectué pendant 700 minutes à 37°C par

$RMN^{31p}$ en prenant un spectre du milieu réactionnel toutes les 10 minutes. On a tracé des courbes du pourcentage de PPi consommé, du rendement d'hydrolyse et du rendement de transfert en fonction du temps pour chaque enzyme utilisée. Les résultats obtenus étaient les suivants :

- Essai A : Phosphatase alcaline extraite de l'intestin de veau (produit commercial Sigma n° P7640), E.C. 3.1.3.1. ; activité spécifique mesurée : 12 U/mg de protéine. Vitesse initiale de disparition du PPi : 0,0150%$s^{-1}$
Vitesse initiale d'hydrolyse : 0,0087%$s^{-1}$
Vitesse initiale de transfert : 0,0062%$s^{-1}$

Remarque : Ces vitesses sont données par la tangente à l'origine de chacune des trois courbes précitées.
Avancement en % des réactions après 700 minutes de réaction à 37°C :
% de PPi consommé : 92,8%
Rendement d'hydrolyse : 39,4%
Rendement de transfert : 53,4%

- Essai B : phosphatase alcaline d'Escherichia coli (produit commercial Sigma n° P4377), E.C. 3.1.3.1.; activité spécifique mesurée : 217 U/mg de protéine. Vitesse initiale de disparition du PPi : 0,0055%$s^{-1}$
Vitesse initiale d'hydrolyse : 0,0038%$s^{-1}$
Vitesse initiale de transfert : 0,0017%$s^{-1}$

Avancement en % des réactions après 700 minutes de réaction à 37°C :
% de PPi consommé : 57,1%
Rendement d'hydrolyse : 28,4%
Rendement de transfert : 28,7%

- Essai C : phosphatase alcaline extraite de l'intestin de poulet (produit commercial Sigma n° P8008), E.C. 3.1.3.1. ; activité spécifique mesurée : 15 U/mg de protéine. Vitesse initiale de disparition du PPi : 0,0022%$^{-1}$
Vitesse initiale d'hydrolyse : 0,0015%$s^{-1}$
Vitesse initiale de transfert : 0,0007%$s^{-1}$

Avancement en % des réactions après 700 minutes de réaction à 37°C.
% de PPi consommé : 6,9%
Rendement d'hydrolyse : 3,4%
Rendement de transfert : 3,5%

Remarques :

Dans chacun des différents essais, on note la présence d'un signal minoritaire en $RMN^{31p}$ correspondant à l'isomère β-glycérol phosphate (glycérol phosphorylé en position 2) mais qui ne représente jamais plus de 5% du rendement de transfert.

Les résultats obtenus pour cette étude montrent que l'avancement de la réaction est différent en fonction de l'enzyme et ils mettent en évidence l'activité importante de la phosphatase alcaline extraite de l'intestin de veau par rapport à la phosphatase alcaline d'Escherichia coli et à la phosphatase alcaline extraite de l'intestin de poulet.

Par ailleurs, la vitesse initiale de transfert est quatre fois plus grande quand on utilise la phosphatase alcaline extraite de l'intestin de veau par rapport à la réaction utilisant la phosphatase alcaline d'Escherichia coli, alors que la vitesse initiale de transfert dans le cas de la phosphatase alcaline extraite de l'intestin de poulet reste très faible.

De plus, le rapport du rendement de transfert sur celui de l'hydrolyse après 700 minutes de réaction est de 1,35 dans le cas de la phosphatase alcaline extraite de l'intestin de veau, alors qu'il n'est que de 0,99 pour la phosphatase alcaline d'Escherichia coli et de 0,97 pour la phosphatase alcaline extraite de l'intestin de poulet.

Ces résultats démontrent la supériorité surprenante de la phosphatase alcaline d'intestin de veau.

Exemple 3

On a étudié l'influence de la pureté de la phosphatase alcaline d'intestin de veau en utilisant des conditions expérimentales similaires à celles décrites pour l'exemple 2, y compris l'emploi de 350 unités d'enzyme pour 3 ml de milieu réactionnel. Les résultats obtenus étaient les suivants :

- Essai A de l'exemple 2 (pour mémoire) : phosphatase alcaline extraite de l'intestin de veau (produit commercial Sigma n° P7640), E.C.3.1.3.1 ; activité spécifique mesurée : 12 U/mg de protéine. Vitesse initiale de disparition du PPi : 0,0150%s$^{-1}$
Vitesse initiale d'hydrolyse : 0,0087%s$^{-1}$
Vitesse initiale de transfert : 0,0062%s$^{-1}$

Avancement en % des réactions après 700 minutes de réaction à 37°C.
% de PPi consommé : 92,8%
Rendement d'hydrolyse : 39,4%
Rendement de transfert : 53,4%

- Essai D : phosphatase alcaline extraite de l'intestin de veau (produit commercial Boehringer n° 108154), E.C. 3.1.3.1. ; activité spécifique mesurée : 80 U/mg de protéine. Vitesse initiale de disparition du PPi : 0,0125%s$^{-1}$
Vitesse initiale d'hydrolyse : 0,0072%s$^{-1}$
Vitesse initiale de transfert : 0,0052%s$^{-1}$

Avancement en % des réaction après 700 minutes de réaction à 37°C.
% de PPi consommé : 83,3%
Rendement d'hydrolyse : 34,3%
Rendement de transfert : 49,0%

Les résultats obtenus montrent que l'avancement de la réaction est assez peu sensible à la pureté de l'enzyme et que la vitesse initiale de la réaction de transfert de phosphate est du même ordre de grandeur dans les deux cas. Un léger avantage existe, toutefois, en faveur de l'enzyme la moins pure de l'essai A.

Exemple 4 Influence du type de phosphate utilisé comme donneur.

On a étudié l'influence du type de polyphosphate utilisé comme donneur en utilisant des conditions expérimentales similaires à celles décrites à l'exemple 2, mais en faisant varier le polyphosphate utilisé. A cet effet, on a essayé divers polyphosphates ayant la formule générale

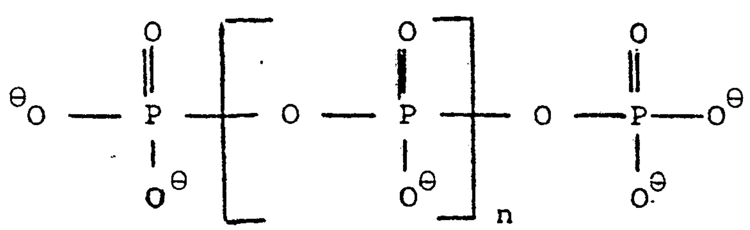

avec n = 0, 1, 3, 13, 23, 43 et 73. On a déterminé le pourcentage de polyphosphate consommé, le rendement d'hydrolyse et le rendement de transfert au bout de 48 heures de réaction et au bout de 91 heures de réaction. Les résultats obtenus sont récapitulés ci-dessous :

Au bout de 48 heures :

| Polyphosphate utilisé | % de phosphate consommé | Rendement d'hydrolyse | Rendement de transfert |
|---|---|---|---|
| n = 0 (PPi) | 90,6% | 48,4% | 42,2% |
| n = 1 | 51,4% | 16,0% | 35,4% |
| n = 3 | 38,8% | 9,8% | 29,0% |
| n =13 | 8,4% | 1,4% | 7,0% |
| n =23 | 6,8% | 3,4% | 3,4% |
| n =43 | 4,8% | 1,1% | 3,7% |
| n =.73 | 2,8% | 0,56% | 2,24% |

Tous les polyphosphates utilisés sont hydrolysés par l'enzyme et, dans tous les cas, on a un transfert de phosphate sur le glycérol ; cependant, le % de polyphosphate consommé et les rendements de transfert et d'hydrolyse diminuent quand la longueur de la chaîne du polyphosphate augmente.

Au bout de 91 heures

| Polyphosphate utilisé | % de phosphate consommé | Rendement d'hydrolyse | Rendement de transfert |
|---|---|---|---|
| n = 0 (PPi) | 100,00% | 24,80% | 75,20% |
| n = 1 | 88,00% | 24,00% | 64,00% |
| n = 3 | 63,70% | 20,40% | 43,30% |
| n = 13 | 16,00% | 6,00% | 10,00% |
| n = 23 | 6,60% | 1,95% | 4,50% |
| n = 43 | 6,40% | 1,90% | 4,50% |
| n = 73 | 2,60% | 0,37% | 2,23% |

Après 43 heures supplémentaires de réaction, on a une augmentation du % de polyphosphate consommé mais surtout une augmentation très importante du rendement de transfert pour le PPi et les polyphosphates à courte chaîne.

Une telle augmentation du rendement de transfert ne peut s'expliquer que par une phosphorylation du glycérol à partir du phosphate formé précédemment dans le milieu. En effet, les 9,4% de PPi restant au bout de 48 heures de réaction ne sont pas suffisants pour expliquer une telle augmentation du rendement de transfert.

A la suite de ces derniers résultats, on a procédé à des essais comparatifs de phosphorylation du glycérol à l'aide de phosphate de sodium (Pi) et de pyrophosphate de sodium (PPi) comme agents de phosphorylation en utilisant des conditions expérimentales semblables à celles de l'exemple 2, si ce n'est qu'on a conduit la réaction pendant 2h 40 minutes en prenant un spectre de $RMN^{31p}$ du milieu réactionnel toutes les 10 minutes.

Les résultats obtenus sont consignés dans le tableau suivant :

| Temps de réaction (minutes) | Donneur de phosphate | | | |
|---|---|---|---|---|
| | Pyrophosphate PPi | | | Phosphate Pi |
| | % de PPi consommé | Rendement d'hydrolyse | Rendement de transfert | Rendement de transfert |
| 0 | 0% | 0% | 0% | 0% |
| 20 | 10,0% | 6,0% | 4,0% | 0% |
| 30 | 16,4% | 10,2% | 6,2% | 0% |
| 40 | 22,5% | 12,5% | 10,0% | 1,0% |
| 60 | 32,0% | 19,0% | 13,0% | 3,1% |
| 80 | 38,0% | 21,5% | 16,5% | 4,5% |
| 100 | 43,8% | 24,0% | 19,8% | 5,5% |
| 120 | 49,0% | 26,0% | 23,0% | 8,0% |
| 140 | 53,5% | 29,0% | 24,5% | 10,4% |
| 160 | 56,0% | 30,8% | 25,2% | 11,2% |

Ces essais montrent que le phosphate Pi est transféré aussi sur le glycérol. Toutefois, les résultats ci-dessus et les études cinétiques faites conjointement auxdits essais montrent que :

- la vitesse initiale de transfert est 3,5 fois plus élevée en utilisant le PPi comme donneur,
- et, surtout, la présence d'une phase de latence de 30 minutes dans le cas du Pi alors qu'aucune phase de latence n'est observée dans le cas du PPi.

Les résultats obtenus dans cette étude montrent très clairement que le transfert du résidu phosphate est favorisé lorsqu'on utilise le PPi comme donneur à la place du Pi.

Exemple 5 Préparation de monophosphate d'éthylène-glycol à partir d'éthylène-glycol.

En utilisant des conditions opératoires similaires à celles de l'exemple 1 sauf que l'on a remplacé le glycérol par de l'éthylène-glycol utilisé en des proportions allant de 40 à 90% en volume du milieu réactionnel, on a pu obtenir la production de monophosphate d'éthylène-glycol. Au bout de 48 heures de réaction, on a mesuré le pourcentage de PPi consommé, le rendement d'hydrolyse et le rendement de transfert. Les résultats obtenus sont récapitulés dans le tableau suivant:

| % en volume d'éthylène glycol | % de PPi consommé | Rendement d'hydrolyse | Rendement de transfert |
|---|---|---|---|
| 40% | 100,0% | 65,7% | 34,3% |
| 50% | 100,0% | 59,7% | 40,3% |
| 60% | 100,0% | 41,5% | 58,5% |
| 70% | 48,6% | 26,2% | 22,4% |
| 80% | 6,2% | 3,9% | 2,3% |
| 90% | 3,7% | 2,5% | 1,2% |

On voit qu'entre 40% et 60% d'éthylèneglycol, on a 100% de PPi consommé, et on remarque que le rendement d'hydrolyse diminue au fur et à mesure que la concentration en éthylèneglycol augmente.

Le rendement de transfert est bon entre 40 et 70% d'éthylène-glycol et passe par un maximum à 58,5% pour une concentration de 60% en éthylèneglycol.

Exemple 6 Phosphorylation d'autres composés hydroxylés

Afin de démontrer que le procédé de l'invention peut s'appliquer à la phosphorylation de divers types de composés hydroxylés, on a conduit une série d'essais en utilisant les conditions opératoires de l'exemple 1 mais en substituant au glycérol une proportion semblable d'un autre composé hydroxylé. Les composés hydroxylés utilisés, le pourcentage de PPi consommé et les rendements d'hydrolyse et de transfert obtenus sont indiqués dans le tableau ci-après.

| Composé hydroxylé | % de PPi consommé | Rendement de transfert | Rendement d'hydrolyse |
|---|---|---|---|
| **Sucres** | | | |
| D-Glucose | 82,0 % | 15,2 % | 66,8 % |
| D-Ribose | 66,0 % | 9,4 % | 56,6 % |
| **Alcools** | | | |
| $CH_3$-CHOH-C≡CH | 83,5 % | 12,1 % | 71,4 % |
| $CH_2$=CH-$CH_2$ OH | 53,5 % | 9,8 % | 43,7 % |
| **Diols et polyols** | | | |
| HO $CH_2$-CHOH-$CH_2$OH | 100,0 % | 51,4 % | 48,6 % |
| HO $CH_2$-$CH_2$ OH | 56,4 % | 19,5 % | 36,9 % |
| HO $CH_2$-CHOH-$CH_3$ | 61,2 % | 15,6 % | 45,6 % |
| HO $CH_2$-$CH_2$-$CH_2$OH | 62,2 % | 20,2 % | 42,0 % |
| HO $CH_2$-$CH_2$-O($CH_2$-$CH_2$)$_2$-O$CH_2$-$CH_2$OH | 66,9 % | 11,4 % | 55,5 % |
| HO $CH_2$-$CH_2$-O-$CH_2$-$CH_2$ OH | 53,2 % | 16,6 % | 36,6 % |
| $CH_3$-CHOH-CHOH-$CH_3$ | 71,0 % | 12,2 % | 58,8 % |
| HO ($CH_2$-$CH_2$-O-)$_2$-$CH_2$-$CH_2$OH | 60,5 % | 11,4 % | 49,1 % |
| HO$CH_2$-CHOH-$CH_2$-$CH_2$OH | 100,0 % | 52,1 % | 47,9 % |
| HO$CH_2$-$CH_2$-$CH_2$-$CH_2$OH | 58,0 % | 18,7 % | 39,3 % |
| HO$CH_2$-$CH_2$-$CH_2$-$CH_2$-$CH_2$OH | 64,5 % | 29,5 % | 35,2 % |
| HO$CH_2$-CH=CH-$CH_2$OH | 78,8 % | 26,0 % | 52,8 % |
| HO$CH_2$-$CH_2$-S-$CH_2$-$CH_2$-OH | 86,0 % | 31,3 % | 54,7 % |
| HO$CH_2$-CHOH-CHOH-CHOH-CHOH-$CH_2$OH | 91,3 % | 21,1 % | 70,2 % |

Il est à noter que ces essais ont été conduits simplement pour démontrer la faisabilité du procédé de l'invention, mais qu'on n'a pas cherché à optimiser les conditions opératoires.

Il va de soi que les modes de réalisation décrits ne sont que des exemples et qu'on pourrait les modifier, notamment par substitution d'équivalents techniques, sans sortir pour cela du cadre de l'invention.

## Revendications

1. Un procédé de préparation de dérivés phosphatés de composés hydroxylés caractérisé en ce qu'on effectue une réaction de phosphorylation enzymatique mettant en jeu un composé organique hydroxylé, un donneur de phosphate qui est un pyrophosphate ou polyphosphate de la formule :

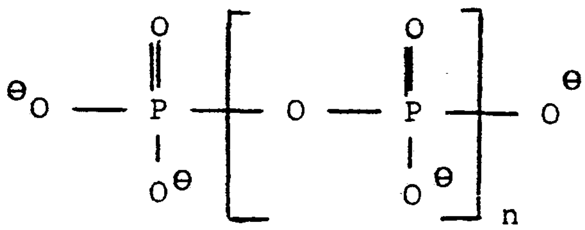

où n = 0 à 74, et
de la phosphatase alcaline d'intestin de veau, à un pH de 4,5 à 9.

2. Un procédé selon la revendication 1, caractérisé en ce que le pH est compris entre 5,3 et 8,0.

3. Un procédé selon la revendication 1 ou 2, caractérisé en ce qu'on utilise un donneur de phosphate de la formule indiquée dans laquelle n = 1 à 4.

4. Un procédé selon la revendication 3, caractérisé en ce qu'on utilise un pyrophosphate (n = 1) comme donneur de phosphate.

5. Un procédé selon l'une quelconque des revendications 1 à 4, caractérisé en ce qu'on utilise une proportion de composé organique hydroxylé comprise entre 40 et 70% en volume par rapport au volume du milieu réactionnel.

6. Un procédé selon l'une quelconque des revendications 1 à 5, caractérisé en ce que le composé organique hydroxylé est choisi parmi les alcools, les diols, les polyols, les oses, les dérivés d'oses, les peptides et les protéines.

7. Un procédé selon la revendication 6, caractérisé en ce que le composé organique hydroxylé est le glycérol ou l'éthylène-glycol.

Office européen des brevets

# RAPPORT DE RECHERCHE EUROPEENNE

Numero de la demande

EP 88 40 1016

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 151 320 (UNILEVER)<br>* Revendications * | 1 | C 12 P 9/00<br>C 12 N 9/16 |
| D,A | CHEMICAL ABSTRACTS, vol. 87, no. 3, 18 juillet 1977, page 240, résumé no. 18192v, Columbus, Ohio, US; K. MARTINEK et al.: "Preparative enzymic synthesis in biphasic aqueous-organic system", & BIOORG. KHIM. 1977, 3(5), 696-702 | 1 | |
| D,A | JOURNAL OF BIOLOGICAL CHEMISTRY, vol. 242, no. 1, 10 janvier 1967, pages 114-119, American Society of Biological Chemists, Inc., Baltimore, MD., US; W.B. ANDERSON et al.: "Inorganic pyrophosphate-glucose phosphotransferase activity associated with alkaline phosphatase of Escherichia coli" | 1 | |
| | | | **DOMAINES TECHNIQUES RECHERCHES (Int. Cl.4)** |
| | | | C 12 P<br>C 12 N |

Le présent rapport a été établi pour toutes les revendications

| Lieu de la recherche | Date d'achèvement de la recherche | Examinateur |
|---|---|---|
| LA HAYE | 03-08-1988 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)